# EUROPEAN PATENT APPLICATION

(11) **EP 4 632 079 A1**
(43) Date of publication of application: **15.10.2025**
(21) Application number: 24169255.7
(22) Date of filing: 09.04.2024
(51) Int. Cl.: C12Q 1/6883

(54) **METHODS OF DETERMINING THE RISK TO DEVELOP COMPLICATIONS RELATED TO ALLO-HSCT**

(71) Applicant: Ecole Polytechnique Fédérale de Lausanne (EPFL), 1015 Lausanne (CH)
(72) Inventor: MARTINS, Filipe, 1224 Chêne-Bougeries (CH); TRONO, Didier, 1134 Vufflens-le-Château (CH); ANSARI, Marc, 1231 Conches (CH); PLANET LETSCHERT, Evarist, 1112 Echichens (CH)
(74) Representative: KATZAROV S.A.

(57) **Abstract**

The present invention provides methods of determining the risk to develop complications related to allogeneic hematopoietic stem cell transplantation (allo-HSCT) in a biological sample isolated from a patient transplanted with allo-HSCT. The invention further provides therapeutic agents, kits and methods of treatment and/prevention of complications related to allo-HSCT.

## Description

### FIELD OF THE INVENTION

The present invention provides methods of determining the risk to develop complications related to allogeneic hematopoietic stem cell transplantation (allo-HSCT) in a biological sample isolated from a patient transplanted with allogeneic hematopoietic stem cells. The invention further provides therapeutic agents, kits and methods of treatment and/prevention of complications related to allo-HSCT.

### BACKGROUND OF THE INVENTION

Allogeneic hematopoietic stem cell transplantation (allo-HSCT) is a potentially curative therapy for patients with malignant and nonmalignant diseases. Despite considerable progress in many areas, acute graft-versus-host disease (aGVHD), a dreadful complication of allo-HSCT, continues to be a serious cause of morbidity and mortality after allogeneic HSCT.
aGVHD affects nearly half of allo-HSCT recipients and is caused by a rejection of their skin, gut, and liver tissues by the donor T-cells.

Despite preventive interventions, the onset of aGVHD is often rapid and its evolution to severe forms is difficult to predict, therefore enforcing the need for designing early diagnostic tools and new therapeutic strategies for treating and/or preventing complications related to allo-HSCT.

### SUMMARY OF THE INVENTION

The present invention provides a method of determining the risk to develop complications related to allogeneic hematopoietic stem cell transplantation (allo-HSCT) in a biological sample isolated from a patient transplanted with allo-HSCT, the method comprising:
i) determining the level of transcription and/or expression and/or activity of the monocyte biomarkers SIGLEC1, CD163, and CCL3; and/or
ii) determining the level of transcription and/or expression and/or activity of the cytotoxic T cell (CTL) biomarkers CXCL10 receptor CXCR3 and CD70;
iii) comparing the level of transcription and/or expression and/or activity of said monocyte and/or CTL biomarkers in the transplanted patient blood sample with the level of transcription and/or expression and/or activity in a control sample or with a predetermined reference level,
wherein an increased level of transcription and/or expression and/or activity of said monocyte and/or CTL biomarkers in the transplanted patient sample compared to the control sample or compared to the predetermined reference level indicates that the patient is at risk to develop complications related to allo-HSCT.

Also provided is a kit for determining the risk to develop complications related to allogeneic hematopoietic stem cell transplantation (allo-HSCT) in a blood sample, the kit comprising means for determining the level of transcription and/or expression and/or activity of the monocyte and/or CTL biomarkers of the invention.

Also provided is the use of a method of the invention, or a kit of the invention, for determining the risk to develop complications related to allogeneic hematopoietic stem cell transplantation (allo-HSCT) in a blood sample isolated from a patient transplanted with allo-HSCT.

Also provided is a device for performing a method according to the invention, said device comprising:
i) a sample chamber for a test sample collected from a patient transplanted with allo-HSCT;
ii) an assay module in fluid communication with said sample chamber, said assay module comprising means and/or reagents for measuring, directly or indirectly, the expression level of transcription and/or expression and/or activity of at least one of the biomarkers of any one of claims 1 to 8 in said test sample;
iii) means for computing an allo-HSCT probability score; and
iv) a user interface wherein said user interface relates the allo-HSCT probability score to detecting the risk to develop complications related to allo-HSCT.

The present invention provides a method of detecting the onset, progression and/ or regression of a complications related to allogeneic hematopoietic stem cell transplantation (allo-HSCT) in a biological sample isolated from a patient transplanted with allo-HSCT, the method comprising:
i) determining the level of transcription and/or expression and/or activity of the monocyte biomarkers SIGLEC1, CD163, and CCL3; and/or
ii) determining the level of transcription and/or expression and/or activity of the cytotoxic T cell (CTL) biomarkers CXCL10 receptor CXCR3 and CD70;
iii) comparing the level of transcription and/or expression and/or activity of said monocyte and/or CTL biomarkers in the transplanted patient blood sample with the level of transcription and/or expression and/or activity in a control sample or with a predetermined reference level,
wherein an increased level of transcription and/or expression and/or activity of said monocyte and/or CTL biomarkers in the transplanted patient sample compared to the control sample or compared to the predetermined reference level is indicative of the onset, progression or regression of said complications related to allo-HSCT.

The present invention provides a method of determining the risk to develop complications related to allogeneic hematopoietic stem cell transplantation (allo-HSCT) in a biological sample isolated from a patient transplanted with allo-HSCT, the method comprising:
i) detecting, directly or indirectly, the relative abundance of one or more atypical cell types and/or measuring the level of transcription, expression and/or activity of one or more atypical cell types in a sample obtained from said subject;
ii) periodically comparing the relative abundance and/or level of transcription, expression and/or activity of said one or more atypical cell types,
wherein an alteration in the relative abundance and/or level of transcription, expression and/or activity of one or more of these atypical cell types in a sample obtained from the same subject, relative to the relative abundance and/or level of transcription, expression and/or activity of one or more of these atypical cell types, determined previously, indicates that the patient is at risk to develop complications related to allo-HSCT.

The present invention provides a method of detecting the onset, progression and/ or regression of a complications related to allogeneic hematopoietic stem cell transplantation (allo-HSCT) in a biological sample isolated from a patient transplanted with allo-HSCT, said method comprising:
i) detecting, directly or indirectly, the relative abundance of one or more atypical cell types and/or measuring the level of transcription, expression and/or activity of one or more atypical cell types in a sample obtained from said subject;
ii) periodically comparing the relative abundance and/or level of transcription, expression and/or activity of said one or more atypical cell types,
wherein an alteration in the relative abundance and/or level of transcription, expression and/or activity of one or more of these atypical cell types in a sample obtained from the same subject, relative to the relative abundance and/or level of transcription, expression and/or activity of one or more of these atypical cell types, determined previously, is indicative of the onset, progression or regression of said complications related to allo-HSCT

The present invention provides an agent modulating the transcription and/or expression and/or activity
- of a monocyte biomarker selected from the group comprising SIGLEC1, CD163, and CCL3, or a combination of two or more thereof; and/or
- a cytotoxic T cell (CTL) biomarker selected from the group comprising CXCL10 receptor CXCR3 and CD70, or a combination thereof.

### BRIEF DESCRIPTION OF THE FIGURES

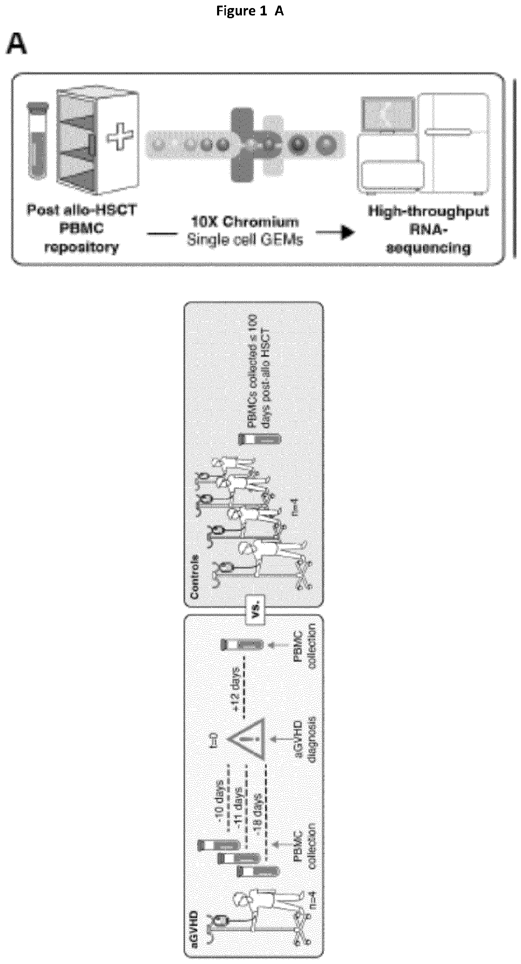

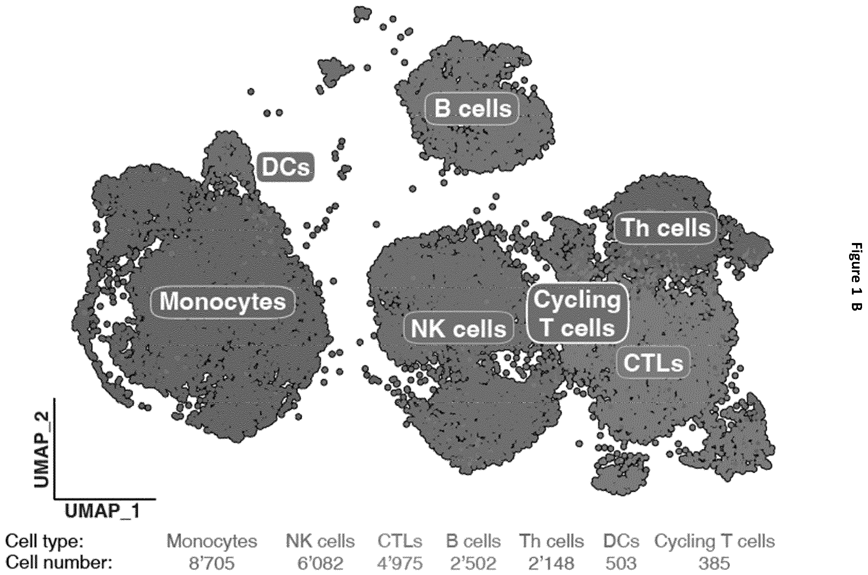

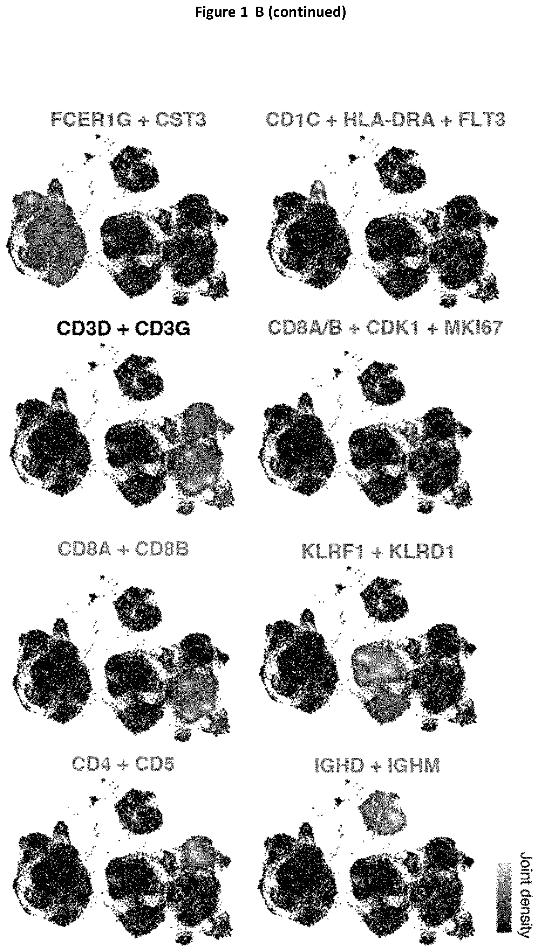

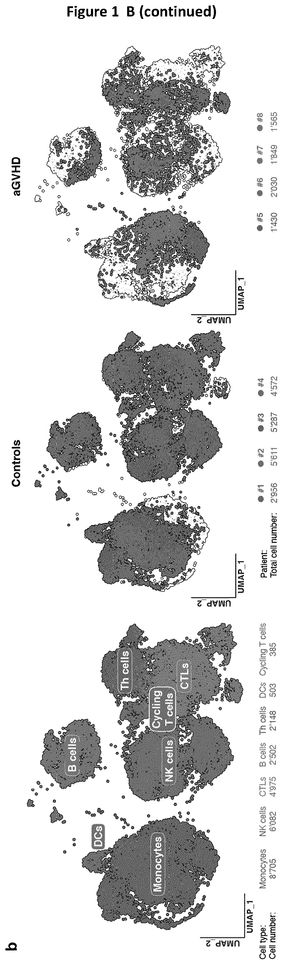

**Figure 1****. Single-Cell Gene Expression Profiling of PBMCs collected after allo-HSCT. (A)** Schematic outline of the study design. Stored PBMCs from eight pediatric patients, including samples from 4 patients who did not experience aGVHD disease during their post-alloHSCT follow-up were subject to scRNA-seq (10X Chromium). **(B)** (Top) Uniform manifold approximation and projection (UMAP) plot showing the single-cell transcriptomes of 22'798 PBMCs issued from all the patients included in this study after removal of dead cells, red blood cells, platelets, and patient-specific cell clusters. The cells are colored according to cell type. (Bottom) UMAP plots showing, for control (left) and pre-/aGVHD (right) cases, the single-cell integration obtained after the projection of disease cells onto control cell clusters. The color code represents the patient origin of cells.

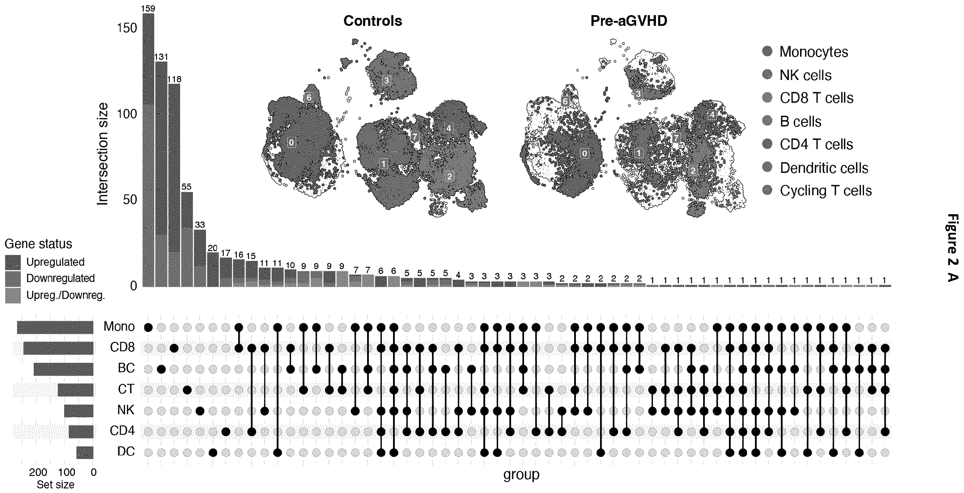

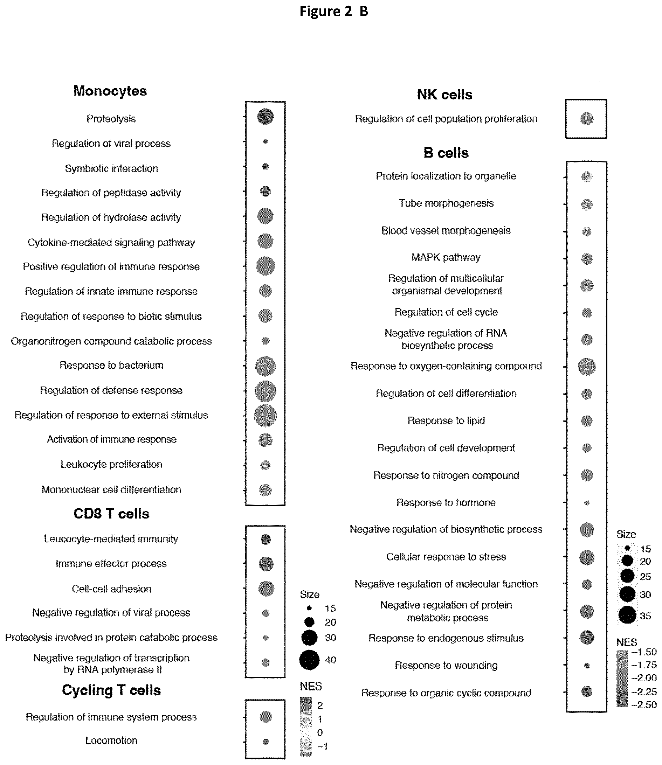

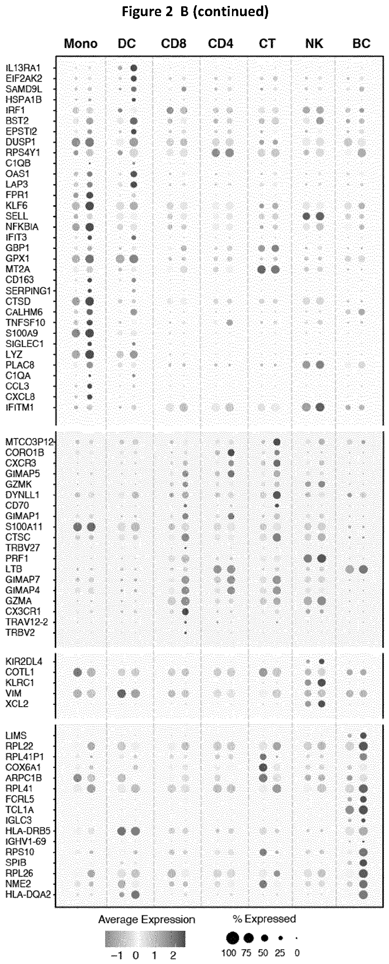

**Figure 2****. Differential gene expression analysis and GSEA of PBMC-derived scRNA-seq data.** (A) (Top) Same UMAP plots as in (Figure 1B, bottom) but with a color code representing cell types as in (1B, top). Upset plot showing unique or overlapping DEGs (FDR <0.05) derived from the comparison between control and pre-/aGVHD samples within each cluster. The black dots represent the DEGs that are shared by more than two cell types. The bar graphs represent the number of DEGs for each category and the side grey bars represent the total number of DEGs between control and pre-/aGVHD groups of each cell type. Different colors represent the number of genes either upregulated, downregulated, or both up-/downregulated in each category. (B) (Left) Dot plots of significantly enriched or depleted gene ontology biological processes in pre-/aGVHD compared to controls. Only cell type-specific gene ontologies are shown. Dot size represents the number of genes in each gene ontology (count) and the color scale represents the normalized enrichment score (NES). (Right) Dot plot showing the significantly upregulated genes (Fold change >2 and FDR <0.05) in monocytes and CTLs, NK cells, and B cells from pre-/aGVHD patients compared to controls. Dot size represents the proportion of cells expressing the gene in each condition and across cell types.

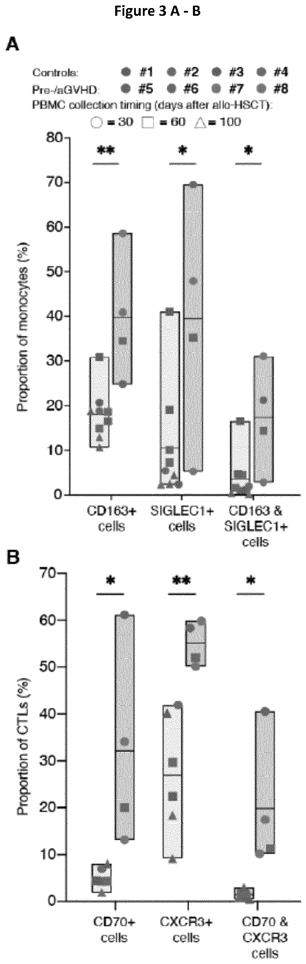

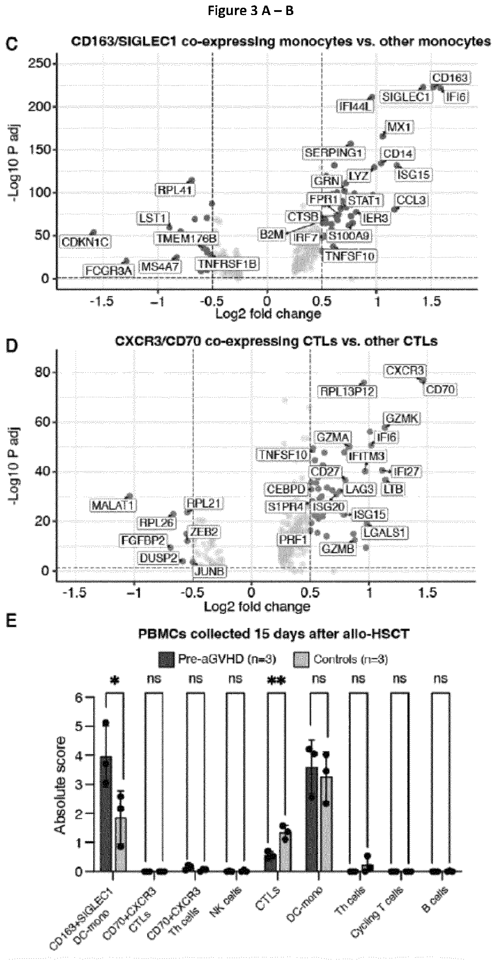

**Figure 3****. The presence of blood circulating CD163/SIGLEC1 monocytes precedes the clinical onset of aGVHD.** (A) Bar plot showing the proportion of monocytes expressing either CD163, SIGLEC1, or both surface marker genes in control (light grey bars) and pre-/aGVHD samples (dark grey bars). Symbols with the same color indicate samples issued from the same patient. Samples with less than 100 monocytes were excluded from this analysis. Statistics: Student's T-test. (B) Bar plot showing the proportion of CTLs expressing either CXCR3, CD70, or both surface marker genes in control (light grey bars) and pre-/aGVHD samples (dark grey bars). Symbols with the same color indicate samples issued from the same patient. Samples with less than 100 CTLs were excluded from this analysis. Statistics: Student's T-test. (C) Volcano plot of DEGs between CD163/SIGLEC1 co-expressing monocytes and monocytes expressing either one or none of these markers. Genes with a Log2 fold change >0.5 and an adjusted P value <0.05 are shown in dark red. (D) Volcano plot of DEGs between CXCR3/CD70 co-expressing CTLs and CTLs expressing either one or none of these markers. Genes with a Log2 fold change >0.5 and an adjusted P value <0.05 are shown in khaki. (E) Bar plot showing the relative abundance of 8 immune cell types in pre-aGVHD (dark grey) and controls (lightgrey) using CIBERSORTx with the gene expression signatures issued from the cell types identified in this study and from the one of the CD163/SIGLEC1 and CXCR3/CD70 co-expressing DC-monocytes and T cells, respectively. Statistics: Student's T-test.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise(s)" and "comprising" also encompass the more restricted ones "consist(s)", "consisting" as well as "consist/consisting essentially of", respectively.

As used in the specification and claims, the singular form "a", "an" and "the" include plural references unless the context clearly dictates otherwise.

As used herein, "at least one" means "one or more", "two or more", "three or more", etc.

As used herein the terms "subject"/"subject in need thereof", or "patient"/"patient in need thereof" are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject according to the invention is a human, most preferably a human transplanted with peripheral hematopoietic stem cells or bone marrow and suffering from complications related to allo-HSCT or a human transplanted with allo-HSCT that might be at risk of suffering from complications related to allo-HSCT. In some aspects, the patient is treated by allo-HSCT because he or she suffers from one or more life-threatening conditions or diseases.

The term "about" particularly in reference to a given quantity, is meant to encompass deviations of plus or minus ten percent (± 10 %). For example, "about 80%" also encompasses a value between 72% and 88%, or "about 100 bases" also encompasses a number of bases between 90 and 110 bases.

The term "treatment" or "treating" means any administration of a composition, pharmaceutical composition, agent, compound, *etc...* of the disclosure to a subject for the purpose of:
(i) inhibiting the disease, that is, arresting the development of clinical symptoms; and/or
(ii) relieving the disease, that is, causing the regression of clinical symptoms.

As used herein, the term "prevention" or "preventing" means any administration of a composition, pharmaceutical composition, agent, compound, *etc...* of the disclosure to a subject for the purpose of:
(i) preventing the disease, that is, causing the clinical symptoms of the disease not to develop.

In the context of the present invention, the disease refers to one or more complications related to allo-HSCT as disclosed herein. In one aspect, the complication related to allo-HSCT is selected from the group comprising Acute Graft versus Host Disease (aGvHD), chronic GvHD and autoimmune disease, or a combination thereof. The term "hematopoietic stem cell transplantation" or "HSCT" has replaced the term "bone marrow transplantation" (BMT) because hematopoietic stem cells can be derived from a variety of sources other than the bone marrow, including the peripheral blood and umbilical cord blood. In the context of the present invention, HSCT and allo- HSCT also cover BMT.

aGvHD is a reaction of donor immune cells against host tissues (Malard F, Holler E, Sandmaier BM, Huang H, Mohty M. Acute graft-versus-host disease. Nat Rev Dis Primers. 2023 Jun 8;9(1):27). Activated donor T cells damage host epithelial cells after an inflammatory cascade. Initially, there is tissue damage due to conditioning that in turn activates the host antigen-presenting cells (APCs) (first phase). Secondly, APCs activate donor T cells, also known as an afferent phase (second phase). Finally, in the efferent phase (third phase), cellular and inflammatory factors work together to damage the target organs (e.g. skin, liver, and gastrointestinal tract).

While profiling the gene expression landscape of peripheral blood mononuclear cells (PBMCs) using single-cell RNA-sequencing (scRNA-seq), the Inventors unveiled the presence of aGVHD-specific monocytes and cytotoxic T cells (CTLs) in patients' blood up to 18 days before clinical disease presentation. These monocytes were characterized by an upregulation of the surface marker CD 163, the transmembrane protein SIGLEC1, and the pro-inflammatory cytokine CCL3, while CTLs were characterized by the upregulation of the CXCL10 receptor CXCR3 and the antigenic stimulation marker CD70.

According to one aspect of the invention, the invention concerns a method of determining the risk to develop complications related to allogeneic hematopoietic stem cell transplantation (allo-HSCT) in a blood sample isolated from a patient transplanted with allo-HSCT, the method comprising:
i) determining the level of transcription and/or expression and/or activity of a monocyte biomarker selected from the group comprising SIGLEC1, CD163, and CCL3, or a combination of two or more thereof; and
ii) comparing the level of transcription and/or expression and/or activity of said biomarker(s) in the transplanted patient blood sample with the level of transcription and/or expression and/or activity in a control sample or with a predetermined reference level.

In one aspect, the combination of two or more monocyte biomarkers consists of a) SIGLEC1, and CD163, b) SIGLEC1 and CCL3, c) CD 163 and CCL3, or d) SIGLEC1, CD 163 and CCL3.

According to one aspect of the invention, the invention concerns a method of determining the risk to develop complications related to allogeneic hematopoietic stem cell transplantation (allo-HSCT) in a blood sample isolated from a patient transplanted with allo-HSCT, the method comprising:
i) determining the level of transcription and/or expression and/or activity of a cytotoxic T cell (CTL) biomarker selected from the group comprising CXCL10 receptor CXCR3 or CD70, or a combination thereof; and
ii) comparing the level of transcription and/or expression and/or activity of said biomarker(s) in the transplanted patient blood sample with the level of transcription and/or expression and/or activity in a control sample or with a predetermined reference level.

According to one aspect of the invention, the invention concerns a method of determining the risk to develop complications related to allogeneic hematopoietic stem cell transplantation (allo-HSCT) in a blood sample isolated from a patient transplanted with allo-HSCT, the method comprising:
i) determining the level of transcription and/or expression and/or activity of a monocyte biomarker selected from the group comprising SIGLEC1, CD163, and CCL3, or a combination of two or more thereof; and/or
ii) determining the level of transcription and/or expression and/or activity of a cytotoxic T cell (CTL) biomarker selected from the group comprising CXCL10 receptor CXCR3 and CD70, or a combination thereof;
   iii) comparing the level of transcription and/or expression and/or activity of said biomarkers in the transplanted patient blood sample with the level of transcription and/or expression and/or activity in a control sample or with a predetermined reference level.

In one aspect, the combination of two or more monocyte biomarkers consists of a) SIGLEC1, and CD 163, b) SIGLEC1 and CCL3, c) CD 163 and CCL3, or d) SIGLEC1, CD 163 and CCL3.

The one or more monocyte biomarkers described herein can be combined with any of the cytotoxic T cell (CTL) biomarkers described herein.

In the context of the invention, an increased level of transcription and/or expression and/or activity of at least one of said biomarkers in the transplanted patient blood sample compared to the control sample or compared to the predetermined reference level indicates that the patient is at risk to develop complications related to allo-HSCT.

Usually, the level of corresponding transcription and/or expression and/or activity level of said biomarkers has been determined previously and corresponds to a control sample, a reference sample, a group of reference samples, or a reference value.

In one aspect, the control sample has been determined in a biological sample of the same patient before being transplanted with allo-HSCT (i.e. control sample or baseline).

In one aspect, the determination or detection has been done in the biological sample between about 1 to about 16 weeks, about 2 to about 14 weeks, about 2 to about 12 weeks, about 2 to about 10 weeks, after the transplantation with allo-HSCT.

In one aspect, the determination or detection has been done about at least 1 month before, about at least 1 week before, about at least one day before, about at least 1 hour, about at least 1 minute before the transplantation with allo-HSCT. Alternatively, the biological sample has been collected before the transplantation with allo-HSCT, but the determination is done after the transplantation with allo-HSCT.

In one aspect, the determination or detection is done periodically, i.e. at least every day, at least every 2 days, at least every 5 days, at least every 10 days, at least every 15 days, at least every 20 days, at least every 30 days, at least every 45 day, at least every 60 days, at least every 70 days, after the transplantation with allo-HSCT.

As used herein, "allo-Hematopoietic stem cell transplantation", abbreviated as "allo-HSCT" refers to a cell therapy that is the most used cell therapy currently. Allo-HSCT is the recommended treatment for several life-threatening conditions or diseases such as leukemia, rare blood cell diseases, aplastic anemia, and to restore the hematopoietic system after bone marrow ablating chemotherapy or irradiation therapy. More than one million allo-HSCT has been done worldwide so far.

Hematopoietic stem cells (HSC) are responsible for the lifelong production of blood cells. The special characteristics of the HSCs, such as self-renewal and multipotency, have guaranteed the success of HSCT. Among others, main bottlenecks of allo-HSCT are HSC donor shortage and allo-HSCT failure by infusing a reduce number of donor HSC. Different approaches have been addressed to solve these problems, such as using different sources of HSCs (bone marrow, cord blood, mobilized peripheral blood, amniotic fluid or placental cord), generating engraftable HSCs from embryonic cells, and expanding HSCs ex vivo.

The biomarkers of the invention are selected from monocyte biomarkers and/or cytotoxic T cell biomarkers.

According to one aspect of the invention, the monocyte biomarker is selected from the group comprising SIGLEC1, CD163 and/or CCL3.

Sialic acid binding Ig like lectin 1 (SIGLEC1; also known as CD169, Siglec1 or Sialoadhesin (Sn)), is a surface adhesion molecule present on human myeloid cells. Being part of the Siglec family, it acts as a receptor for sialylated molecular structures, which are found among various pathogenic and non-pathogenic ligands. Alternatively spliced transcript variants (ENST00000707083.1, ENST00000419548.4 and ENST00000344754.6) encoding different isoforms have been described for this gene and are enbrassed in the present invention.

Several antibodies directed against Human Siglec-1 are commercially available and can be used in the methods described herein. Non limiting examples comprise: His Tag (SG1-H52H3) (ACRO biosystem; see e.g. Guo M, Guo H, Zhu J, Wang F, Chen J, Wan C, Deng Y, Wang F, Xu L, Chen Y, Li R, Liu S, Zhang L, Wang Y, Zhou J, Li S. A novel subpopulation of monocytes with a strong interferon signature indicated by SIGLEC-1 is present in patients with in recent-onset type 1 diabetes. Diabetologia. 2024 Apr;67(4):623-640)); CD169 (Siglec-1) Antibody, anti-human, Vio ^{®} Bright R720, REAfinity^{™} (Miltenyi Biotec); and HSn 7D2 (Novus Biologicals).

CD163 is a surface protein receptor exclusively expressed in monocytes and macrophages. This receptor is encoded by a gene which is a member of the scavenger receptor cysteine-rich (SRCR) superfamily. It functions as an acute phase-regulated receptor involved in the clearance and endocytosis of hemoglobin/haptoglobin complexes by macrophages and may thereby protect tissues from free hemoglobin-mediated oxidative damage. Alternatively spliced transcript variants (ENST00000542705.1, ENST00000542280.5, ENST00000541972.5, ENST00000539632.1, ENST00000538840.1, ENST00000537626.5, ENST00000537044.1, ENST00000534848.1, ENST00000432237.3, ENST00000396620.7, and ENST00000359156.8) encoding different isoforms have been described for this gene and are embraced in the present invention.
Several antibodies directed against Human CD 163 are commercially available and can be used in the methods described herein. Non limiting examples comprise: CoraLite^{®} Plus 488 Anti-Human CD163 (GHI/61) (proteintech); CD163 Monoclonal Antibody (10D6) (InVitrogen); recombinant monoclonal CD163 antibody [EPR19518] and [OTI2G12] (abcam); CD163 Antibody (GHI/61) mouse monoclonal IgG1 κ CD163 antibody (Santa Cruz Biotechnology) and OR2805, a monoclonal antiboby targeting CD163 (see e.g. clinical trial NCT05094804).

CCL3, a member of the chemokine family, is also named macrophage inflammatory protein-1α (MIP-1α) and consists of about 70 amino acids with a molecular weight of about 7 kDa (Menten P, Wuyts A, Van Damme J. Macrophage inflammatory protein-1. Cytokine Growth Factor Rev. 2002 Dec;13(6):455-81.) Alternatively spliced transcript variants (ENST00000613922.2, ENST00000614051.1 and ENST00000613928.1) encoding different isoforms have been described for this gene and are embraced in the present invention.
Several antibodies directed against Human CCL3/MIP-1α are commercially available and can be used in the methods described herein. Non limiting examples comprise: CCL3 (MIP-1α) Antibody, anti-human, REAfinity^{™}. Clone: REA257 (Miltenyi Biotec); Anti-CCL3 Monoclonal (756613) (Catalog # MA5-24364 Invitrogen); and Rabbit recombinant monoclonal MIP-1 alpha/CCL3 antibody [EPR16618-90, Abcam].

According to one aspect of the invention, the cytotoxic T cell biomarker is selected from the group comprising CXCR3 and/or CD70.

Chemokine (C-X-C motif) receptor 3 (CXCR3) and its ligand (CXCL10) regulate immune responses. CXCL10/CXCR3 activation contributes to the pathogenesis of many inflammatory diseases. Alternatively spliced transcript variants (ENST00000373693.4 and ENST00000373691.4) encoding different isoforms have been described for this gene and are embraced in the present invention.
Several antibodies directed against Human CXCL10 receptor CXCR3 are commercially available and can be used in the methods described herein. Non limiting examples comprise: Anti-CXCR3 antibody [EPR25373-32, EPR23845-44, MM0223-7K22, Abcam); and CXCR3 Antibody (5C10E6, Novus Biologicals).

CD70 (Cluster of Differentiation 70) is a 50 kDa type II transmembrane glycoprotein cell surface glycoprotein that in humans is encoded by CD70 gene and known as a ligand for CD27. Expression of CD70 is tightly controlled and upregulated exclusively upon activation on T cells, B cells and certain subsets of dendritic cells (DCs). CD70 expression is very limited in the steady state, although increased levels of CD70 have been reported to be associated with inflammatory conditions such as chronic viral infection, cancer, or autoimmune disease. Alternatively spliced transcript variants (ENST00000245903.4, ENST00000423145.7 and ENST00000597430.2) encoding different isoforms have been described for this gene and are embraced in the present invention.
Several antibodies directed against Human CD70 are commercially available and can be used in the methods described herein. Non limiting examples comprise: CD70 Antibodies (301731, MAB2738, Novus Biologicals); CD70 Antibodies (AF2738, Novus Biologicals); and vorsetuzumab.

The term "level" refers to an absolute quantification of a molecule or an analyte in a sample (e.g. a biomarker), or to a relative quantification of a molecule or analyte in a sample, i.e., relative to another value such as relative to a reference value as taught herein, or to a range of values for the biomarker in the absence of treatment or after starting the treatment. These values or ranges can be obtained from a single patient or alternatively from a group of patients.

Various techniques for determining differential level(s) transcription and/or expression and/or activity of one or more biomarkers are known in the art.

For example, one may calculate differential expression of one biomarker in a test sample by, e.g. calculating the ratio (fold change) between the transcription and/or expression level of the biomarker in the test sample and the expression level of the biomarker in the reference sample or group of samples, or reference value.

Transcription and/or expression level can be measured as transcripts per million (TPM) by RNA seq, as Threshold cycles (Ct) by PCR, as probe fluorescence intensity by microarray, *etc....*

Determining transcriptional changes in a group of samples for all the transcriptome is usually done using computational methods to determine differential gene expression in a full RNAseq dataset (e.g. 15000 genes). Different commonly used methods are, e.g., selected among the following software packages (open source): edgeR, DESeq2, limma, Cuffdiff, PoissonSeq, baySeq, *etc...* Preferably, DESeq2 is used as described in Love, M.I., Huber, W. & Anders, S. Moderated estimation of fold change and dispersion for RNA-seq data with DESeq2. Genome Biol 15, 550 (2014).

The transcripts of the genes encoding the biomarkers of the invention can be detected and, alternatively, quantitated by a variety of methods including, but not limited to, microarray analysis, polymerase chain reaction (PCR), reverse transcriptase polymerase chain reaction (RT-PCR), reverse transcriptase real-time PCR, quantitative real-time PCR, digital PCR, end-point PCR, multiplex end-point PCR, Northern blot, serial analysis of gene expression (SAGE), immunoassay, mass spectrometry, in situ hybridization (ISH), multiplex in situ hybridization, and any RNA sequencing-based methods known in the art such next generation nucleic acid sequencing or a combination of said methods.

Example of next generation nucleic acid sequencing are selected form the group comprising whole transcriptome RNA seq, targeted RNA seq, single cell RNA seq, total RNA sequencing, mRNA sequencing, whole transcript RNA sequencing, bulk-RNA sequencing, 3' RNA sequencing, long RNA sequencing, direct RNA sequencing, or a combination of said methods.

Measurement of the level of **transcription and/or expression of** a biomarker can be direct or indirect. For example, the abundance levels of RNAs can be directly quantitated. Alternatively, the amount of a biomarker can be determined indirectly by measuring abundance levels of cDNAs, amplified RNAs or DNAs, or by measuring quantities or activities of RNAs, or other molecules that are indicative of the expression level of the biomarker (such as, e.g proteins). Preferably, the amount of a biomarker is determined indirectly by measuring abundance levels of cDNAs.

In one aspect, microarrays are used to measure the levels of transcription and/or expression of biomarkers. An advantage of microarray analysis is that the transcription and/or expression of each of the biomarkers can be measured simultaneously, and microarrays can be specifically designed to provide a diagnostic expression profile for a disease or condition or the invention.

Microarrays are prepared by selecting probes which comprise a polynucleotide sequence, and then immobilizing such probes to a solid support or surface. For example, the probes may comprise DNA sequences, RNA sequences, or copolymer sequences of DNA and RNA. The polynucleotide sequences of the probes may also comprise DNA and/or RNA analogues, or combinations thereof. For example, the polynucleotide sequences of the probes may be full or partial fragments of genomic DNA. The polynucleotide sequences of the probes may also be synthesized nucleotide sequences, such as synthetic oligonucleotide sequences. The probe sequences can be synthesized either enzymatically in vivo, enzymatically in vitro (e.g., by PCR), or non-enzymatically in vitro.

Probes used in the methods of the invention are preferably immobilized to a solid support which may be either porous or non-porous. For example, the probes may be polynucleotide sequences which are attached to a nitrocellulose or nylon membrane or filter covalently at either the 3' or the 5' end of the polynucleotide. Such hybridization probes are well known in the art (see, e.g., Sambrook, et al., Molecular Cloning: A Laboratory Manual (3rd Edition, 2001). Alternatively, the solid support or surface may be a glass or plastic surface. In one aspect, hybridization levels are measured to microarrays of probes consisting of a solid phase on the surface of which are immobilized a population of polynucleotides, such as a population of DNA or DNA mimics, or, alternatively, a population of RNA or RNA mimics. The solid phase may be a nonporous or, optionally, a porous material such as a gel.

In one aspect, the microarray comprises a support or surface with an ordered array of binding (e.g., hybridization) sites or "probes" each representing one of the genes described herein. Preferably the microarrays are addressable arrays, and more preferably positionally addressable arrays. More specifically, each probe of the array is preferably located at a known, predetermined position on the solid support such that the identity (i.e., the sequence) of each probe can be determined from its position in the array (i.e., on the support or surface). Each probe is preferably covalently attached to the solid support at a single site.

Microarrays can be made in a number of ways, of which several are described below. However they are produced, microarrays share certain characteristics. The arrays are reproducible, allowing multiple copies of a given array to be produced and easily compared with each other. Preferably, microarrays are made from materials that are stable under binding (e.g., nucleic acid hybridization) conditions. Microarrays are generally small, e.g., between 1 cm2 and 25 cm2; however, larger arrays may also be used, e.g., in screening arrays. Preferably, a given binding site or unique set of binding sites in the microarray will specifically bind (e.g., hybridize) to the product of a single gene in a cell (e.g., to a specific mRNA, RNA, or to a specific cDNA derived therefrom). However, in general, other related or similar sequences will cross-hybridize to a given binding site.

As noted above, the "probe" to which a particular polynucleotide molecule specifically hybridizes contains a complementary polynucleotide sequence. The probes of the microarray typically consist of nucleotide sequences of no more than 1,000 nucleotides. In some aspects, the probes of the array consist of nucleotide sequences of 10 to 1,000 nucleotides. In aspect aspect, the nucleotide sequences of the probes are in the range of 10-200 nucleotides in length and are genomic sequences of one species of organism, such that a plurality of different probes is present, with sequences complementary and thus capable of hybridizing to the genome of such a species of organism, sequentially tiled across all or a portion of the genome. In other aspects, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, or are 60 nucleotides in length. The probes may comprise DNA or DNA "mimics" (e.g., derivatives and analogues) corresponding to a portion of an organism's genome. In another aspect, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone (e.g., phosphorothioates).

DNA can be obtained, e.g., by polymerase chain reaction (PCR) amplification of genomic DNA or cloned sequences. PCR primers are preferably chosen based on a known sequence of the genome that will result in amplification of specific fragments of genomic DNA. Computer programs that are well known in the art are useful in the design of primers with the required specificity and optimal amplification properties, such as Oligo version 5.0 (National Biosciences). Typically each probe on the microarray will be between 10 bases and 50,000 bases, usually between 300 bases and 1,000 bases in length. PCR methods are well known in the art, and are described, for example, in Innis et al., eds., PCR Protocols: A Guide To Methods And Applications, Academic Press Inc., San Diego, Calif. (1990). It will be apparent to one skilled in the art that controlled robotic systems are useful for isolating and amplifying nucleic acids.

An alternative, preferred means for generating polynucleotide probes is by synthesis of synthetic polynucleotides or oligonucleotides, e.g., using N-phosphonate or phosphoramidite chemistries (Froehler et al., Nucleic Acid Res. 14:5399-5407 (1986); McBride et al., Tetrahedron Lett. 24:246-248 (1983)). Synthetic sequences are typically between about 10 and about 500 bases in length, more typically between about 20 and about 100 bases, and most preferably between about 40 and about 70 bases in length. In some aspects, synthetic nucleic acids include non-natural bases, such as, but by no means limited to, inosine. As noted above, nucleic acid analogues may be used as binding sites for hybridization. An example of a suitable nucleic acid analogue is peptide nucleic acid (see, e.g., U.S. Pat. No. 5,539,083).

Probes are preferably selected using an algorithm that takes into account binding energies, base composition, sequence complexity, cross-hybridization binding energies, and secondary structure.

A skilled artisan will also appreciate that positive control probes, e.g., probes known to be complementary and hybridizable to sequences in the target polynucleotide molecules, and negative control probes, e.g., probes known to not be complementary and hybridizable to sequences in the target polynucleotide molecules, should be included on the array. In one aspect, positive controls are synthesized along the perimeter of the array. In another aspect, positive controls are synthesized in diagonal stripes across the array. In still another aspect, the reverse complement for each probe is synthesized next to the position of the probe to serve as a negative control. In yet another aspect, sequences from other species of organism are used as negative controls or as "spike-in" controls.

The probes are attached to a solid support or surface, which may be made, e.g., from glass, plastic (e.g., polypropylene, nylon), polyacrylamide, nitrocellulose, gel, or other porous or nonporous material. One method for attaching nucleic acids to a surface is by printing on glass plates, as known in the art. This method is especially useful for preparing microarrays of cDNA A second method for making microarrays produces high-density oligonucleotide arrays. Techniques are known for producing arrays containing thousands of oligonucleotides complementary to defined sequences, at defined locations on a surface using photolithographic techniques for synthesis in situ (see, U.S. Pat. Nos. 5,578,832; 5,556,752; and 5,510,270) or other methods for rapid synthesis and deposition of defined oligonucleotides. When these methods are used, oligonucleotides (e.g., 60-mers) of known sequence are synthesized directly on a surface such as a derivatized glass slide. Usually, the array produced is redundant, with several oligonucleotide molecules per RNA.

Other methods for making microarrays, e.g., by masking, may also be used. In principle, any type of array known in the art, for example, dot blots on a nylon hybridization membrane could be used. However, as will be recognized by those skilled in the art, very small arrays will frequently be preferred because hybridization volumes will be smaller.

Microarrays can also be manufactured by means of an ink jet printing device for oligonucleotide synthesis, e.g., using the methods and systems described by Blanchard in U.S. Pat. No. 6,028,189;. Specifically, the oligonucleotide probes in such microarrays are synthesized in arrays, e.g., on a glass slide, by serially depositing individual nucleotide bases in "microdroplets" of a high surface tension solvent such as propylene carbonate. The microdroplets have small volumes (e.g., 100 pL or less, more preferably 50 pL or less) and are separated from each other on the microarray (e.g., by hydrophobic domains) to form circular surface tension wells which define the locations of the array elements (i.e., the different probes). The polynucleotide probes are attached to the support covalently at either the 3' or the 5' end of the polynucleotide.

Biomarker which may be measured by microarray analysis can be expressed RNAs or a nucleic acid derived therefrom (e.g., cDNA or amplified RNA derived from cDNA that incorporates an RNA polymerase promoter), including naturally occurring nucleic acid molecules, as well as synthetic nucleic acid molecules. In one aspect, the target polynucleotide molecules comprise RNA, including, but by no means limited to, total cellular RNA, poly(A)+ messenger RNA (mRNA) or a fraction thereof, cytoplasmic mRNA, or RNA transcribed from cDNA (i.e., cRNA; see, e.g., U.S. Pat. No. 5,545,522, 5,891,636, or 5,716,785). Methods for preparing total and poly(A)+ RNA are well known in the art, and are described generally, e.g., in Sambrook, et al., Molecular Cloning: A Laboratory Manual (3rd Edition, 2001). RNA can be extracted from a cell of interest using guanidinium thiocyanate lysis followed by CsCl centrifugation, a silica gel-based column (e.g., RNeasy (Qiagen, Valencia, Calif.) or StrataPrep (Stratagene, La Jolla, Calif.)), or using phenol and chloroform, as known in the art. Poly(A)+ RNA can be selected, e.g., by selection with oligo-dT cellulose or, alternatively, by oligo-dT primed reverse transcription of total cellular RNA. RNA can be fragmented by methods known in the art, e.g., by incubation with ZnCl2, to generate fragments of RNA.

In one aspect, total RNA, mRNAs, or nucleic acids derived therefrom (such as cDNA), are isolated from a sample taken from a patient transplanted with allo-HSCT. Biomarkers that are poorly expressed in particular cells may be enriched using normalization techniques known in the art.

As described above, the biomarker polynucleotides can be detectably labeled at one or more nucleotides. Any method known in the art may be used to label the target polynucleotides. Preferably, this labeling incorporates the label uniformly along the length of the RNA, and more preferably, the labeling is carried out at a high degree of efficiency. For example, polynucleotides can be labeled by oligo-dT primed reverse transcription. Random primers (e.g., 9-mers) can be used in reverse transcription to uniformly incorporate labeled nucleotides over the full length of the polynucleotides. Alternatively, random primers may be used in conjunction with PCR methods or T7 promoter-based in vitro transcription methods in order to amplify polynucleotides.

The detectable label may be a luminescent label. For example, fluorescent labels, bioluminescent labels, chemiluminescent labels, and colorimetric labels may be used in the practice of the invention. Fluorescent labels that can be used include, but are not limited to, fluorescein, a phosphor, a rhodamine, or a polymethine dye derivative. Additionally, commercially available fluorescent labels including, but not limited to, fluorescent phosphoramidites such as FluorePrime (Amersham Pharmacia, Piscataway, N.J.), Fluoredite (Miilipore, Bedford, Mass.), FAM (ABI, Foster City, Calif.), and Cy3 or Cy5 (Amersham Pharmacia, Piscataway, N.J.) can be used. Alternatively, the detectable label can be a radiolabeled nucleotide.

Nucleic acid hybridization and wash conditions are chosen so that the target polynucleotide molecules specifically bind or specifically hybridize to the complementary polynucleotide sequences of the array, preferably to a specific array site, wherein its complementary DNA is located. Arrays containing double-stranded probe DNA situated thereon are preferably subjected to denaturing conditions to render the DNA single-stranded prior to contacting with the target polynucleotide molecules. Arrays containing single-stranded probe DNA (e.g., synthetic oligodeoxyribonucleic acids) may need to be denatured prior to contacting with the target polynucleotide molecules, e.g., to remove hairpins or dimers which form due to self-complementary sequences.

Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook, et al., Molecular Cloning: A Laboratory Manual (3rd Edition, 2001) . Typical hybridization conditions for the cDNA microarrays of Schena et al. are hybridization in 5×SSC plus 0.2% SDS at 65° C. for four hours, followed by washes at 25° C. in low stringency wash buffer (1×SSC plus 0.2% SDS), followed by 10 minutes at 25° C. in higher stringency wash buffer (0.1×SSC plus 0.2% SDS). Particularly preferred hybridization conditions include hybridization at a temperature at or near the mean melting temperature of the probes (e.g., within 51° C., more preferably within 21° C.) in 1 MNaCl, 50 mM MES buffer (pH 6.5), 0.5% sodium sarcosine and 30% formamide.

When fluorescently labeled gene products are used, the fluorescence emissions at each site of a microarray may be, preferably, detected by scanning confocal laser microscopy. In one aspect, a separate scan, using the appropriate excitation line, is carried out for each of the two fluorophores used. Alternatively, a laser may be used that allows simultaneous specimen illumination at wavelengths specific to the two fluorophores and emissions from the two fluorophores can be analyzed simultaneously. Arrays can be scanned with a laser fluorescent scanner with a computer-controlled X-Y stage and a microscope objective. Sequential excitation of the two fluorophores is achieved with a multi-line, mixed gas laser and the emitted light is split by wavelength and detected with two photomultiplier tubes. Fluorescence laser scanning devices are known in the art. Alternatively, a fiber-optic bundle, may be used to monitor mRNA abundance levels at a large number of sites simultaneously.

As discussed above, many RNA sequencing-based methods are available. Each of these sequencing technologies have their own way of preparing samples prior to the actual sequencing step. Depending on the sequencing technology used, amplification steps may be omitted.

According to one aspect, differential expression and/or activity determination of one or more biomarkers of the invention is done at the protein level.

In such cases, various techniques for determining differential expression and/or activity of one or more biomarkers are known in the art.

For example, one may calculate differential expression of one biomarker in a test sample by, e.g. calculating the ratio (fold change) between the expression level of the biomarker in the test sample and the expression level of the biomarker in the reference sample or group of samples, or reference value.

In methods of the invention, the determination may be performed by measuring the concentration of the peptide biomarker in a sample or samples. Biological samples that may be tested in a method of the invention include cerebrospinal fluid (CSF), whole blood, blood serum, plasma, urine, saliva, bone marrow, or other bodily fluid (stool, tear fluid, synovial fluid, sputum), breath, e.g. as condensed breath, or an extract or purification therefrom, or dilution thereof. Biological samples also include tissue homogenates, tissue sections and biopsy specimens from a live subject, or taken post-mortem. The samples can be prepared, for example where appropriate diluted or concentrated, and stored in the usual manner.

In one aspect, the sample is blood, preferably whole blood or a fractional component thereof.

The determination of differential expression and/or activity of biomarker may be direct (e.g. by SELDI or MALDI-TOF) or indirect via interaction with a ligand or ligands such as an antibody or a biomarker-binding fragment thereof, or other peptide, or ligand, e.g. aptamer, or oligonucleotide, capable of specifically binding the biomarker. The ligand may possess a detectable label, such as a luminescent, fluorescent or radioactive label, and/or an affinity tag.

For example, determining the level of expression and/or activity of a peptide biomarker of the invention can be performed by one or more method(s) selected from the group comprising SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC, flow cytometry, CyTOF, any protein/antibody-based detection methods and other LC or LC MS-based techniques. Appropriate LC MS techniques include ICAT^{®} (Applied Biosystems, CA, USA), or iTRAQ^{®} (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

According to one aspect of the invention, the level of transcription and/or expression and/or activity of a biomarker of the invention may be expressed as a score. The score may be calculated as the mean, or the median, or the ratio or the sum, or the weighted mean, median or the sum, the ratio of the expression levels of the genes composing the panel in control samples and disease samples.

Usually, an increased level of transcription and/or expression and/or activity of at least one biomarker of the invention in the transplanted patient sample corresponds to an increase equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 % when compared to previous determinations, control samples or reference levels.

Usually, a decreased level of transcription and/or expression and/or activity of at least one biomarker of the invention in the transplanted patient sample corresponds to a decrease equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 % when compared to previous determinations, control samples or reference levels.

The present invention also provides a method of detecting the onset, progression and/ or regression of complications related to allogeneic hematopoietic stem cell transplantation (allo-HSCT) in a biological sample isolated from a patient transplanted with allo-HSCT, the method comprising:
i) determining the level of transcription and/or expression and/or activity of a monocyte biomarker selected from the group comprising SIGLEC1, CD163, and CCL3, or a combination of two or more thereof; and
ii) comparing the level of transcription and/or expression and/or activity of said biomarker(s) in the transplanted patient blood sample with the level of transcription and/or expression and/or activity in a control sample or with a predetermined reference level.
In one aspect, the combination of two or more monocyte biomarkers consists of a) SIGLEC1, and CD163, b) SIGLEC1 and CCL3, c) CD163 and CCL3, or d) SIGLEC1, CD163 and CCL3.

According to one aspect of the invention, the invention concerns a method of detecting the onset, progression and/ or regression of complications related to allogeneic hematopoietic stem cell transplantation (allo-HSCT) in a blood sample isolated from a patient transplanted with allo-HSCT, the method comprising:
i) determining the level of transcription and/or expression and/or activity of a cytotoxic T cell (CTL) biomarker selected from the group comprising CXCL10 receptor CXCR3 or CD70, or a combination thereof; and
ii) comparing the level of transcription and/or expression and/or activity of said biomarker(s) in the transplanted patient blood sample with the level of transcription and/or expression and/or activity in a control sample or with a predetermined reference level.

According to one aspect of the invention, the invention concerns a method of detecting the onset, progression and/ or regression of complications related to allogeneic hematopoietic stem cell transplantation (allo-HSCT) in a blood sample isolated from a patient transplanted with allo-HSCT, the method comprising:
i) determining the level of transcription and/or expression and/or activity of a monocyte biomarker selected from the group comprising SIGLEC1, CD163, and CCL3, or a combination of two or more thereof; and/or
ii) determining the level of transcription and/or expression and/or activity of a cytotoxic T cell (CTL) biomarker selected from the group comprising CXCL10 receptor CXCR3 and CD70, or a combination thereof;
iii) comparing the level of transcription and/or expression and/or activity of said biomarkers in the transplanted patient blood sample with the level of transcription and/or expression and/or activity in a control sample or with a predetermined reference level.

In one aspect, the combination of two or more monocyte biomarkers consists of a) SIGLEC1, and CD163; b) SIGLEC1 and CCL3, CD163 and CCL3, c) as well as SIGLEC1, CD163 and CCL3 .

In one aspect, an increased level of transcription and/or expression and/or activity of said monocyte and/or CTL biomarkers in the transplanted patient sample compared to the control sample or compared to the predetermined reference level indicates that the patient is at risk to develop complications related to allo-HSCT or that the complications related to allo-HSCT are progressing.

In one aspect, a decreased level of transcription and/or expression and/or activity of said monocyte and/or CTL biomarkers in the transplanted patient sample compared to the control sample or compared to the predetermined reference level indicates that the complications related to allo-HSCT are regressing.

In one aspect, a decreased level of transcription and/or expression and/or activity of said monocyte and/or CTL biomarkers in the transplanted patient sample compared to the control sample or compared to the predetermined reference level is indicative of the responsiveness of the subject to a treatment and/or prevention (such as e.g. prophylaxis), preferably to a treatment and/or prevention of one or more complications related to allo-HSCT.

Treatments and/or prophylaxis of one or more complications related to allo-HSCT comprise: an effective amount of an anti-IL-22 antibody, an anti-IL-6 antibody, donor-type CX3CR1hi MNPs, donor-type NK cells, a ceacam-1 antagonist, an anti-Gr-1 antibody, or a combination thereof, corticosteroids are used for initial treatment of aGVHD. Some patients develop steroid-resistant or refractory (SR) gut-aGVHD (see e.g. Zeiser R, Blazar BR. Acute Graft-versus-Host Disease - Biologic Process, Prevention, and Therapy. N Engl J Med. 2017 Nov 30;377(22):2167-2179; Malard F, Holler E, Sandmaier BM, Huang H, Mohty M. Acute graft-versus-host disease. Nat Rev Dis Primers. 2023 Jun 8;9(1):27.)

Usually, the level of corresponding transcription and/or expression and/or activity level of said biomarkers has been determined previously and corresponds to a control sample, a reference sample, a group of reference samples, or a reference value.

In one aspect, the control sample has been determined in a biological sample of the same patient before being transplanted with allo-HSCT (i.e. control sample or baseline).

In one aspect, the determination or detection has been done in the biological sample between about 1 to about 16 weeks, about 2 to about 14 weeks, about 2 to about 12 weeks, about 2 to about 10 weeks, after the transplantation with allo-HSCT.

In one aspect, the determination or detection has been done about at least 1 month before, about at least 1 week before, about at least one day before, about at least 1 hour, about at least 1 minute before the transplantation with allo-HSCT. Alternatively, the biological sample has been collected before the transplantation with allo-HSCT, but the determination is done after the transplantation with allo-HSCT.

In one aspect, the determination or detection is done periodically, i.e. at least every day, at least every 2 days, at least every 5 days, at least every 10 days, at least every 15 days, at least every 20 days, at least every 30 days, at least every 45 day, at least every 60 days, at least every 70 days, after the transplantation with allo-HSCT.

The present invention also provides a method of detecting the onset, progression and/or regression of complications related to allo-HSCT (e.g. aGVHD) in a post-allogeneic stem cell transplant subject, said method comprising:
(a) detecting the relative abundance of one or more atypical cell types in a sample obtained from said subject;
(b) periodically comparing the relative abundance of said one or more atypical cell types,
wherein an alteration in the relative abundance of one or more of these atypical cell types in a sample obtained from the same subject, relative to the relative abundance of one or more of these atypical cell types, determined previously, is indicative of the onset, progression and/or regression of said complications related to allo-HSCT.

In one aspect, an alteration in the relative abundance of one or more of these atypical cell types in a sample obtained from the same subject, relative to the relative abundance of one or more of these atypical cell types, determined previously, is indicative of the responsiveness of the subject to a treatment and/or prevention (e.g. prophylaxis).

In the context of the present invention, atypical cell types refer to "atypical monocytes" and/or "atypical cytotoxic T cells".

"Atypical monocytes" refer to monocytes presenting the co-expression of biomarkers correlated with complications related to allo-HSCT such as, e.g. with aGVHD occurrence and presenting a mixed phenotype of M1/M2 either at the gene and/or protein expression level. M1 meaning that they are pro-inflammatory and M2 that they are immunosuppressive, a definition proposed by Mills et al in The Journal of Immunology in 2000 (M-1/M-2 Macrophages and Th1/Th2 Paradigm). In one aspect, these atypical monocytes express a biomarker selected from the group comprising SIGLEC1, CD163, and CCL3, or a combination of two or more thereof such as, e.g. i) SIGLEC1 and CD163, ii) SIGLEC1 and CCL3, iii) CD163 and CCL3, or iv) SIGLEC1, CD163 and CCL3. In one aspect, these atypical monocytes express all three biomarkers, namely SIGLEC1, CD163 and CCL3.

"Atypical cytotoxic T cells" refer to cytotoxic T cells presenting the co-expression of biomarkers correlated with complications related to allo-HSCT such as, e.g. with aGVHD occurrence. In one aspect, these atypical monocytes express a biomarker selected from the group comprising the CXCL10 receptor CXCR3 linked to tissue homing and migration, and the co-stimulator CD70, which serves as a biomarker of antigen priming, or a combination thereof. In one aspect, these atypical monocytes express two biomarkers namely CXCL10 receptor CXCR3 and CD70.

In one aspect, the determination or comparison is done periodically, i.e. at least every day, at least every 2 days, at least every 5 days, at least every 10 days, at least every 15 days, at least every 20 days, at least every 30 days, at least every 45 day, at least every 60 days, at least every 70 days, after the transplantation with allo-HSCT.

There are multiple methods available to detect the biomarkers present on atypical cells within a population of cells. Non-limiting methods include flow cytometry, fluorescence-activated cell sorting (FACS), SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC, flow cytometry, CyTOF, any protein/antibody-based detection methods and other LC or LC MS-based techniques. Appropriate LC MS techniques include ICAT^{®} (Applied Biosystems, CA, USA), or iTRAQ^{®} (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used or any other protein/antibody-based detection methods (for a review, see e.g. Stack, E. C., Wang, C., Roman, K. A., & Hoyt, C. C. (2014). Multiplexed immunohistochemistry, imaging, and quantitation: a review, with an assessment of Tyramide signal amplification, multispectral imaging and multiplex analysis. Methods, 70(1), 46-58; Maecker, H. T., McCoy, J. P., & Nussenblatt, R. (2012). Standardizing immunophenotyping for the Human Immunology Project. Nature Reviews Immunology, 12(3), 191-200.; Pachón, G., Caragol, I. & Petriz, J. Subjectivity and flow cytometric variability. Nat Rev Immunol 12, 396 (2012)).

Also provided is a method of determining if a subject suffering from complications related to allogeneic hematopoietic stem cell transplantation (allo-HSCT) is responsive to a treatment, said method comprising:
(a) detecting the relative abundance of one or more atypical cell types in a sample obtained from said subject;
(b) periodically comparing the relative abundance of said one or more atypical cell types,
wherein an alteration in the relative abundance of one or more of these atypical cell types in a sample obtained from the same subject, relative to the relative abundance of one or more of these atypical cell types, determined previously, is indicative of the responsiveness of the subject to a treatment.

Preferably, the subject is a post-allogeneic stem cell transplant subject.

In one aspect, the determination or comparison is done periodically, i.e. at least every day, at least every 2 days, at least every 5 days, at least every 10 days, at least every 15 days, at least every 20 days, at least every 30 days, at least every 45 day, at least every 60 days, at least every 70 days, after the transplantation with allo-HSCT or the start of the treatment.

In one aspect, the present invention provides an agent modulating the transcription and/or expression and/or activity:
- of a monocyte biomarker selected from the group comprising SIGLEC1, CD163, and CCL3, or a combination of two or more thereof;
- a cytotoxic T cell (CTL) biomarker selected from the group comprising CXCL10 receptor CXCR3 and CD70, or a combination thereof and/or
- an atypical cell of the invention.

In one aspect, the agent is for use in the treatment and/or prevention of a disease related to allo-HSCT, or complications related to allo-HSCT, as described herein.

The terms "agent modulating", "agent of the invention" and "modulator" are used interchangeably.

In a further aspect, the agent is for use in the treatment and/or prevention of a disease related to allo-HSCT or complications related to allo-HSCT, preferably when the level of transcription and/or expression and/or activity of at least one of the biomarkers of the invention is increased.

The agent is usually selected from the group comprising a nucleic acid, a chemical compound, a peptide or analog thereof, an antibody or an antigen-binding fragment thereof, an antibody-drug conjugate, and an antibody mimetic, or a combination of one or more thereof.

Where the agent is a nucleic acid, it will be selected from the group comprising an siRNA, an shRNA, a piRNA, an snRNA, an siRNA capable of interfering the expression of short hairpin (sh), a guide RNA, a nucleic acid including an antisense oligonucleotide, a modified antisense oligonucleotide (e.g. a GapmeR) or a combination of one or more thereof.

The terms "siRNA" and "short interfering RNA" as used herein are interchangeable and refer to single-stranded or double-stranded RNA molecules that are capable of inducing RNA interference. SiRNA molecules typically have a duplex region that is between 18 and 30 base pairs in length.

The terms "piRNA" and "Piwi-interacting RNA" are interchangeable and refer to a class of small RNAs involved in gene silencing. PiRNA molecules typically are between 26 and 31 nucleotides in length.

The term "shRNA" as used herein refers to a nucleic acid molecule comprising at least two complementary portions hybridized or capable of specifically hybridizing to form a duplex structure sufficiently long to mediate RNAi (typically between 15-29 nucleotides in length), and at least one single-stranded portion, typically between approximately 1 and 10 nucleotides in length that forms a loop connecting the ends of the two sequences that form the duplex.

The terms "snRNA" and "small nuclear RNA" are interchangeable and refer to a class of small RNAs involved in a variety of processes including RNA splicing and regulation of transcription factors. The subclass of small nucleolar RNAs (snoRNAs) is also included. The term is also intended to include artificial snRNAs, such as antisense derivatives of snRNAs comprising antisense sequences .

The terms "guide RNA" and "gRNA" are interchangeable and refer to a class of RNAs that specifically recognize the target DNA region of interest and directs nuclease (e.g. Cas nuclease) there for editing. The gRNA is made up of two parts: crispr RNA (crRNA), a 17-20 nucleotide sequence complementary to the target DNA, and a tracr RNA, which serves as a binding scaffold for the nuclease.

Non-limiting examples of modified ASOs include the GapmeRs. As used herein, a GapmeR is a chimeric antisense oligonucleotide that contains a central block of deoxynucleotide monomers sufficiently long to induce RNase H cleavage. Modified ASOs such as modified GapmeRs are also encompassed in the present invention and comprise, e.g. GapmeRs with fixed chemical modification architectures selected from the group comprising i) a gapmer with five 2'-O-methoxyethyl (MOE) modifications in each flank, and a central gap of 10 unmodified dans (e.g. 5-10-5 MOE design), and ii) a gapmer employing three or four locked nucleic acid (LNA) modifications in each flank (e.g. 3-10-3 or 4-8-4 LNA designs), as well as a combination of one or more thereof.

In some aspects, the agent of the invention is an antibody or an antigen-binding fragment thereof.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, and chimeric antibody, humanized antibody as long as these molecules are still biologically active and still bind to at least one peptide of the invention. Preferably the antibody is a monoclonal antibody. Preferably also the monoclonal antibody will be selected from the group comprising the IgG1, IgG2, IgG2a, IgG2b, IgG3 and IgG4 or a combination thereof. Most preferably, the monoclonal antibody is selected from the group comprising the IgG1, IgG2, IgG2a, and IgG2b, or a combination thereof.

An "antigen binding fragment" comprises a portion of a full-length antibody. Examples of antigen binding fragments include Fab, Fab', F(ab')2, and Fv fragments; diabodies; linear antibodies; single-chain antibody molecules; and multispecific antibodies (e.g. bispecific antibodies) formed from antibody fragments.

The antibody or antigen-binding fragment thereof of the invention is typically designed to be uniquely and selectively recognizing and binding at least one biomarker of the invention, in particular if the biomarker is a protein expressed on the surface of the monocyte or CTL.

Non-limiting examples of antibody or antigen-binding fragments thereof of the invention are described herein.

As used herein, a "chemical agent" is a compound that produces change by virtue of its chemical composition and its effects on living tissues and organisms. The chemical agent may be a small molecule inhibitor (SMI), most preferably a non-peptidyl SMI, that has cytotoxic effect on the targeted cells like in the case of an antibody-drug conjugate where the drug is a chemotherapy or another molecule like for examples steroids.

The present invention also contemplates a gene delivery vector, preferably in the form of a plasmid or a vector, that comprises one or more nucleic acid(s) encoding an agent of the invention. Preferably, said agent is a nucleic acid selected from the group comprising an siRNA, an shRNA, an snRNA, a piRNA, an siRNA capable of interfering the expression of short hairpin (sh), a guide RNA, and a nucleic acid including an antisense oligonucleotide, a modified antisense oligonucleotide (e.g. a GapmeR) or a combination of one or more thereof.

As used herein, a "vector" is capable of transferring nucleic acid sequences to target cells (e.g. monocytes or CTL) and may be selected from the group comprising e.g., viral vectors, non-viral vectors, particulate carriers, nano-delivery systems (such as LNP) and liposomes.

Suitable vectors include derivatives of SV40 and known bacterial plasmids, e. g., E. coli plasmids col El, pCR1, pBR322, pMB9 and their derivatives, plasmids such as RP4; phage

DNAs, e. g., the numerous derivatives of phage X, e. g., NM989, and other phage DNA, e. g., Ml 3 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Various viral vectors are used for delivering nucleic acid to cells *in vitro* or *in vivo.* Non-limiting examples are vectors based on Herpes Viruses, Pox- viruses, Adeno-associated virus, Lentivirus, and others. In principle, all of them are suited to deliver an expression cassette comprising an expressible nucleic acid molecule that codes for an agent of the invention. In a preferred aspect, said viral vector is an adenoviral vector, preferably a replication competent adenovirus.

It will be appreciated that in the present method the modification following the introduction of the gene delivery vector (plasmid or vector), or the one or more nucleic acid(s) encoding the agent of the invention, to a host cell (e.g. monocyte or CTL) may occur *ex vivo* or *in vitro,* for instance in a cell culture and in some instances not *in vivo.* In other aspects, it may occur *in vivo.*

The host cell(s) (e.g. single cell or population of cells) of the invention is then reintroduced into the patient in need thereof by any route of administration and/or delivery methods known in the art, as described herein.

The present invention further contemplates a host cell (e.g. single cell or population of cells) comprising, or modified by the introduction of, a gene delivery vector of the invention or a nucleic acid of the invention. In one aspect, the cell is a monocyte or a Cytotoxic T cell (CTL).

Also contemplated in the present invention are compositions and pharmaceutical compositions.

In one aspect, the composition comprises i) an agent of the invention, ii) a gene delivery vector of the invention, or iii) a host cell of the invention.

In one aspect, the pharmaceutical composition comprises a therapeutically effective amount of i) agent of the invention, ii) a gene delivery vector of the invention, or iii) a host cell of the invention, in combination with pharmaceutically acceptable carriers, diluents and/or adjuvants.

The term "therapeutically effective amount" as used herein means an amount of an agent of the invention, ii) a gene delivery vector of the invention, or iii) a host cell of the invention, high enough to significantly positively modify the symptoms and/or condition to be treated, but low enough to avoid serious side effects (at a reasonable risk/benefit ratio), within the scope of sound medical judgment. The therapeutically effective amount of an agent of the invention, ii) a gene delivery vector of the invention, or iii) a host cell of the invention is selected in accordance with a variety of factors including type, species, age, weight, sex and medical condition of the patient; the severity of the condition to be treated; the route of administration; the renal and hepatic function of the patient.

"Pharmaceutically acceptable carrier and/or diluent" means a carrier and/or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients may also be part of the pharmaceutical composition and include starch, glucose, lactose, sucrose, sodium stearate, glycerol monostearate, talc, sodium chloride, dried skim milk, glycerol, propylene glycol, water, ethanol and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

Further contemplated are methods of treatment and/or prevention of one or more complications related to allo-HSCT as disclosed herein. In one aspect, the complication related to allo-HSCT is Acute Graft versus Host Disease (aGvHD) which is an immunologic complication of allogeneic hematopoietic cell transplantation.

In one aspect, the method of treatment and/or prevention of one or more complications related to allo-HSCT comprises administering a therapeutically effective amount of an agent modulating the transcription and/or expression and/or activity of a biomarker, or of the atypical cells described herein.

In another aspect, the method of treatment and/or prevention of one or more complications related to allo-HSCT comprises administering a pharmaceutical composition described herein.

In another aspect, the method of treatment and/or prevention of one or more complications related to allo-HSCT comprises administering an agent described herein modulating the expression and/or activity of i) atypical monocytes, and/or ii) atypical cytotoxic T cells, of the invention to a subject in need thereof.

In some aspect, the methods of treatment described above further comprise the administration of an additional therapy, preferably the additional therapy is selected from the group comprising an autoimmune disease therapy and cancer therapy.

Examples of additional therapies comprise administering an effective amount of: an anti-IL-22 antibody, an anti-IL-6 antibody, donor-type CX3CR1hi MNPs, donor-type NK cells, a ceacam-1 antagonist, an anti-Gr-1 antibody, corticosteroids or a combination thereof.

Also contemplated in the present invention are kits for implementing the methods described herein.

In one aspect, the present invention provides a kit for determining the risk to develop complications related to allogeneic hematopoietic stem cell transplantation (allo-HSCT) in a blood sample, the kit comprising means for determining the level of transcription and/or expression and/or activity of at least one of the biomarkers described herein.

Also provided is the use of the method for determining the risk to develop complications related to allogeneic hematopoietic stem cell transplantation (allo-HSCT) of the invention, the biomarkers of the invention, or the kit described herein, for determining the risk to develop complications related to allogeneic hematopoietic stem cell transplantation (allo-HSCT) in a blood sample isolated from a patient transplanted with allo-HSC10.

Also provided are methods of treatment and/or prevention of one or more complications related to allo-HSCT.

In one aspect, the method of treatment and/or prevention of one or more complications related to allo-HSCT comprises administering i) a pharmaceutical composition of the invention ii) a therapeutically effective amount of an agent of the invention, iii) a therapeutically effective amount of a gene delivery vector of the invention, and/or iv) a a therapeutically effective amount of a host cell of the invention.

Also provided is a device for performing a method of determining the risk to develop complications related to allogeneic hematopoietic stem cell transplantation (allo-HSCT) according to the invention, said device comprising:
i) a sample chamber for a test sample collected from a patient transplanted with allo-HSCT;
ii) an assay module in fluid communication with said sample chamber, said assay module comprising means and/or reagents for measuring, directly or indirectly, the expression level of transcription and/or expression and/or activity of at least one of the biomarkers of the invention in said test sample;
iii) means for computing an allo-HSCT probability score; and
iv) a user interface wherein said user interface relates the allo-HSCT probability score to detecting the risk to develop complications related to allo-HSCT.

Also provided is a computer-implemented method for implementing the method for performing a method according to the invention in a patient transplanted with allo-HSCT, said computer-implemented method comprising
i) measuring, directly or indirectly, the expression level of transcription and/or expression and/or activity of at least one of the biomarkers of the invention in a test sample;
ii) calculating a probability score based on the measurement of step (i), and
iii) comparing the probability score calculated in step (ii) with a pre-determined reference score associated to a known risk to develop complications related to allo-HSCT,
wherein a positive deviation of the probability score indicates that the patient is at risk of developing complications related to allo-HSCT.

The invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

### EXAMPLES

### Peripheral blood mononuclear cell (PBMC) collection

PBMCs were isolated from Sodium Heparine anticoagulated venous blood of post allo-HSCT acute lymphoblastic leukemia (ALL) pediatric patients using CPT^{™} Mononuclear Cell Preparation Tubes (from BD Vacutainer^{®} Cat# 362780). Density gradient separation of mononuclear cells from whole blood was performed in accordance with the manufacturer's instructions. PBMCs were resuspended and aliquoted in Recovery^{™} Cell Culture Freezing Medium (Themo Scientific Cat# 12648010) prior freezing with CoolCell^{®} Alcohol-Free Cell Freezing Containers (Azenta Cat# BCS-405) at -80°C for 24h before final liquid nitrogen freezing.

### Single-cell RNA-seq sample processing

PBMCs were thawed in a 37°C water bath for 2 min, transferred into 15 ml tubes and washed in 8 ml of pre-warmed 1X phosphate-buffered saline supplemented with 2% FCS, centrifuged at 250 × g for 10 min. The supernatant was removed, and the cell pellet was then resuspended in 2 mL 1X PBS containing 2% FCS. Dead cells were removed by magnetic bead purification using the EasySep^{™} Dead Cell Removal (Annexin V) Kit (STEMCELL technologies) according to manufacturer's instructions. After isolation, the cells were diluted at a concentration of 500-1000 cells/ml in PBS 2% FCS and checked for viability and absence of significant doublets or aggregates. The cells were then loaded into a Chromium Single Cell Controller (10x Genomics, Pleasanton, CA) in a NextGEM chip K (PN-1000286) together with beads, master mix reagents (containing RT enzyme and poly-dt RT primers) and oil to generate single-cell-containing droplets. Single-cell Gene Expression libraries were then prepared using Chromium Next GEM Single Cell 5' Kit v2.0 (PN-1000263) following the manufacturer's instruction (protocol CG000331 Rev A). Quality control was performed with a TapeStation 4200 (Agilent) and QuBit dsDNA high sensitivity assay (Thermo) following manufacturer instructions. With this procedure, the cDNAs from distinct droplets harbor a distinct and unique 10x "cell barcode".

The sequencing libraries were loaded onto an Illumina HiSeq 4000 paired-end Flow Cell and sequenced at ca 50k reads/cell using read lengths of 26 nt for read1 and 90 nt for read2. The Cell Ranger Single Cell Software v5.0.0 was used to perform sample demultiplexing, barcode processing, and 5' gene counting using default parameters and the human genome assembly GRCh 19.

### Single-cell RNA-seq data processing and analysis

Cell Ranger was used to obtain gene counts on individual cells from FASTQ files. The GRCh19 human genome was used as a reference. Cells with less than 200 features detected or with more than 25% of the reads being assigned to Mitochondria were removed. Seurat's SCTransform function was used to normalize the data using the proportion of mitochondrial reads as a covariate. Seurat's IntegrateData function was used to project each disease sample into the space of the healthy samples. Clusters of cells were defined with the FindCluster function of Seurat. Cell type annotation was conducted using the marker genes issued from the "FindAllMarkers" function in Seurat R package. The following genes were used for cell type annotation: CD3G, CD3D, CD3E (T cells); CD8A, CD8B (cytotoxic T cells); CD4, CD5 (helper T cells), KLRF1, KLRD1 (NKs); IGHD, IGHM (B cells); FCER1G, CST3 (Monocytes); CD1C, HLA-DRA, FLT3 (dendritic cells); CD8A, CD8B, MKI67, CDK1 (cycling cytotoxic T cells); PPBP, GP9, PF4 (Megakaryocytes/Platelets). Differential expression was done with Seurat's FindMarkers function. Genes were considered to be differentially expressed when they had an FDR below 0.05 unless otherwise specified. Graphs were obtained by GraphPad Prism software version 9 or ggplot2 R package.

### Results

Leveraging PBMCs preserved in a biobank from pediatric recipients of allo-HSCT, we conducted a comparative analysis of their transcriptome. Specifically, we compared PBMCs issued from 4 patients collected either before (n=3) or after (n=1) the onset of aGVHD with control samples obtained from 4 patients (n=9) with no history of aGVHD (Figure 1A/B; cf. Table 1 for patient characteristics). These samples were collected at day 30 (n=5), 60 (n=5), and 100 (n=3) after allo-HSCT as part of the biobank protocol.

Our investigation revealed that the vast majority of changes in gene expression were cell-type specific (Figure 2A). Notably, monocytes exhibited the most substantial number of differentially expressed genes (DEG) between pre-/aGVHD and controls. A gene set enrichment analysis (GSEA)⁸ (Figure 2B) revealed that genes associated with proteolysis (e.g., CTSD, LAP3), the complement system (C1QA, SERPING1), cytokine production (TNFSF10, CCL3, CXCL8), interferon response (IFIT3, OAS1, IFITM1), chemotaxis (FPR1), and the calprotectin gene component (S100A9) were upregulated in monocytes, whereas in CD8 T cells the expression of genes linked to tissue homing and migration, including CX3CR1, S100A11, and the CXCL10 receptor CXCR3, as well as cytolytic genes (GZMA, GZMK, PRF1), and the co-stimulator CD70, which serves as a marker of antigen priming⁹, was increased .

Interestingly, circulating monocytes in the pre-/aGVHD patient group exhibited an upregulation of the gene encoding the M2 polarization marker CD163 (Figure 2B), a transmembrane protein involved in the clearance of hemoglobin-haptoglobin complexes¹⁰, and of *SIGLEC1*, which codes for CD169, a surface adhesion molecule for sialylated structures involved in antigen transfer²¹. CD163 and *SIGLEC1* co-expressing cells were also overrepresented in pre-/aGHVD (Figure 3A) and were characterized by an upregulation of IFN-responsive genes (*IFI44L, ISG15, IFI6*)*,* the transcription factor-encoding *STAT1*, and the cytokine genes *CCL3*, and *TNFSF10*, the products of which have been involved in CD8 T cell recruitment²² and reported to contribute to the graft-versus-tumor effect²³, respectively. These cells also displayed downregulation of mononuclear cell differentiation markers such as *TMEM176B²⁴*, *MS4A7²⁵* and *FCGR3A* (*CD16*), while upregulating *CD14* (Figure 3C). Similarly, CTLs co-expressing *CXCR3* and *CD70* were overrepresented amongst pre-/aGVHD samples (Figure 3B) and were marked by the upregulation of cytolytic genes (*GZMA, GZMB, GZMK, PRF1*)*,* IFN-responsive genes (*IFITM3, IFI27, ISG20, IFI6*), the transcription factor-encoding *CEBPD*, and the cytokine gene *TNFSF10* (Figure 3D), thus pointing to an activated T cell population poised for tissue homing.

To validate these findings, we utilized publicly available bulk RNA-seq data of PBMCs from 6 patients collected 15 days after allo-HSCT²⁶, 3 of whom were diagnosed with aGVHD several days later. By deconvoluting this data using the transcriptomic signature of *CD163*/*SIGLEC1* co-expressing mononuclear phagocytes (DC-mono), *CXCR3*/*CD70* T-lymphocytes and of the other cell types identified in our dataset, we were able to infer that *CD163*/*SIGLEC1* co-expressing mononuclear phagocytes were indeed increased in those patients (Figure 2D). However, we did not detect an increase in *CXCR3*/*CD70* co-expressing CTLs in this dataset, possibly due to the lower numbers of CTLs in its pre-aGVHD samples.

CD163+ macrophages are recognized for their anti-inflammatory properties and play a role in tissue repair after injury, potentially leading to scarring and fibrosis in cases of excessive activation^{11,12,13}. However, their increased presence in various inflammatory diseases suggests that they may not solely represent a marker of chronic inflammation but may actively contribute to pathogenesis¹⁴. Furthermore, the measurement of soluble CD163 (sCD163) has been found to correlate with disease activity in multiple autoimmune diseases^{15,16,17}. Some studies have also linked CD163+ macrophages infiltration to cutaneous and gut disease severity and resistance to steroid therapy in aGVHD^{18,19,20}.

These results warrant evaluating the diagnostic value of CD163⁺/CD169⁺ monocytes as an early biomarker of aGVHD. This could easily be tested by performing flow cytometry or cytometry by time of flight (CyTOF) on PBMCs during the high-risk period after allo-HSCT. Our data also point to a so far underappreciated role for circulating CD163 monocytes in the development of aGVHD development. This has important therapeutic implications, as it suggests that anti-CD163 monoclonal antibodies coupled with steroids, which are clinically approved but have not been tested in this indication, might help prevent the occurrence of aGVD in allo-HSCT recipients.

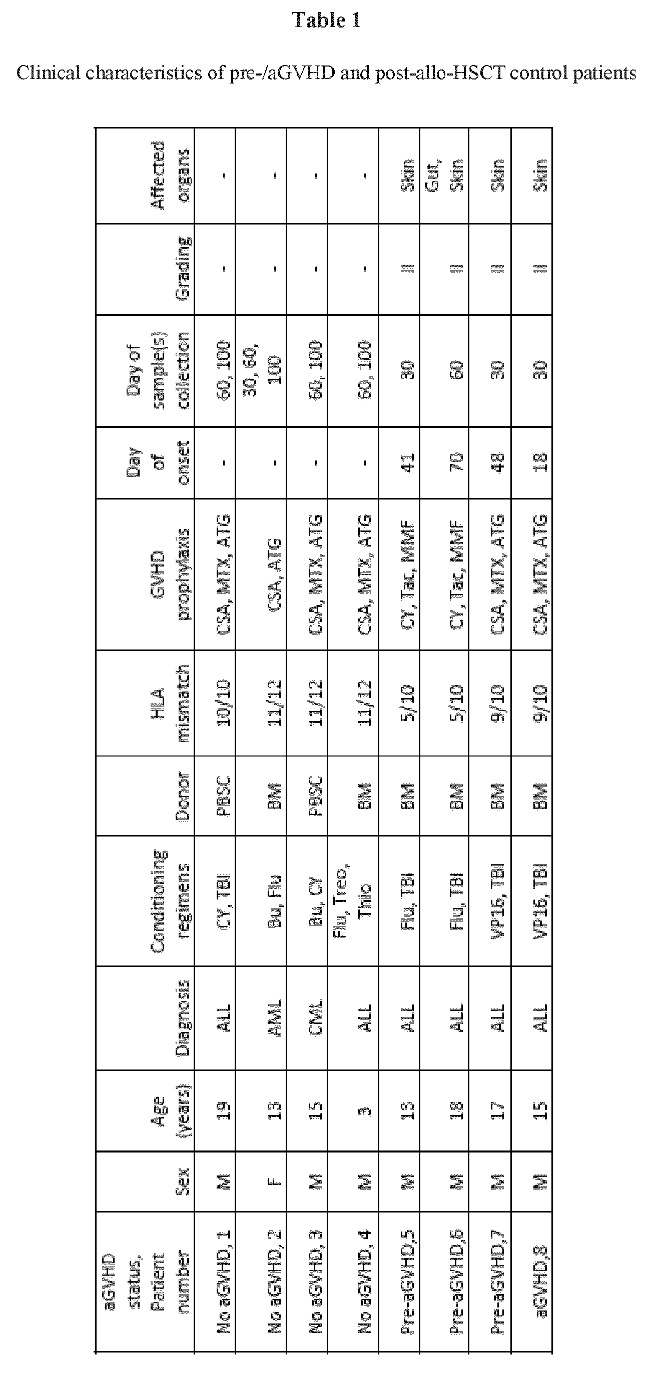

**Abbreviations:** M, Male; F, Female; ALL, acute lymphoblastic leukemia, AML, acute myeloid leukemia; CML, chronic myelogenous leukemia; CY, cyclophosphamide; TBI, total body irradiation; Bu, busulfan; Flu, fludarabine; Treo, treosulfan; Thio, thiotepa; VP16, etoposide; PBSC, peripheral blood stem cell; BM, bone marrow; CSA, ciclosporine A; MTX, methotrexate; ATG, anti-thymocyte globulin; Tac, tacrolimus; MMF, mycophenolate mofetil.

### References

1. Atarod S, Ahmed MM, Lendrem C, et al. miR-146a and miR-155 Expression Levels in Acute Graft-Versus-Host Disease Incidence. Front Immunol. 2016;7. doi:10.3389/fimmu.2016.00056
2. Hartwell MJ, Özbek U, Holler E, et al. An early-biomarker algorithm predicts lethal graft-versus-host disease and survival. JCI Insight. 2017;2(3). doi:10.1172/jci.insight.89798
3. Skytthe MK, Graversen JH, Moestrup SK. Targeting of CD163+ Macrophages in Inflammatory and Malignant Diseases. IJMS. 2020;21(15):5497.
4. Van Halteren AGS, Suwandi JS, Tuit S, et al. A unique immune signature in blood separates therapy-refractory from therapy-responsive acute graft-versus-host disease. Blood. 2023;141(11):1277-1292.
5. Nishiwaki S, Terakura S, Ito M, et al. Impact of macrophage infiltration of skin lesions on survival after allogeneic stem cell transplantation: a clue to refractory graft-versus-host disease. Blood. 2009;114(14):3113-3116.
6. Inamoto Y, Martin PJ, Paczesny S, et al. Association of Plasma CD163 Concentration with De Novo-Onset Chronic Graft-versus-Host Disease. Biol Blood Marrow Transplant. 2017;23:1250-1256.
7. Stary, G. (2023). Good or bad cops: immune cells in aGVHD. Blood 141, 1238-1240. 10.1182/blood.2022018226.
8. Muto, Y., Fujimura, T., Kambayashi, et al. The significance of M1 - polarized CD163 + macrophages in acute graft - versus - host disease (GVHD): Possible mechanisms of GVHD in the development of skin lesions. J Cutaneous Imm & Allergy 6, 120-124. 10.1002/cia2.12304.
9. Duffner U, Lu B, Hildebrandt GC, Teshima T, Williams DL, Reddy P, Ordemann R, Clouthier SG, Lowler K, Liu C, Gerard C, Cooke KR, Ferrara JL. Role of CXCR3-induced donor T-cell migration in acute GVHD. Exp Hematol. 2003 Oct;31(10):897-902. doi: 10.1016/s0301-472x(03)00198-x.

## Claims

1. A method of determining the risk to develop complications related to allogeneic hematopoietic stem cell transplantation (allo-HSCT) in a biological sample isolated from a patient transplanted with allo-HSCT, the method comprising:
i) determining the level of transcription and/or expression and/or activity of the monocyte biomarkers SIGLEC1, CD163, and CCL3; and/or
ii) determining the level of transcription and/or expression and/or activity of the cytotoxic T cell (CTL) biomarkers CXCL10 receptor CXCR3 and CD70;
iii) comparing the level of transcription and/or expression and/or activity of said monocyte and/or CTL biomarkers in the transplanted patient blood sample with the level of transcription and/or expression and/or activity in a control sample or with a predetermined reference level,
wherein an increased level of transcription and/or expression and/or activity of said monocyte and/or CTL biomarkers in the transplanted patient sample compared to the control sample or compared to the predetermined reference level indicates that the patient is at risk to develop complications related to allo-HSCT.

2. The method of claim 1, wherein the complications related to allo-HSCT are selected from the group comprising Acute graft-versus-host disease (aGVHD), chronic GVHD and autoimmune diseases.

3. The method of claim 1 or 2, wherein the increased level of transcription and/or expression and/or activity of said monocyte and/or CTL biomarkers in the transplanted patient sample corresponds to an increase equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 %.

4. The method of any one of the preceding claims, wherein the biological sample is blood and/or bone marrow.

5. The method of claim 4, wherein blood is whole blood or a fractional component thereof.

6. The method of claim 5, wherein the fractional component of the blood sample is white blood cells or peripheral blood mononuclear cells (PBMC).

7. The method of any one of the preceding claims, wherein the level of transcription and/or expression is measured by microarray expression profiling, PCR, reverse transcriptase PCR, reverse transcriptase real-time PCR, quantitative real-time PCR, digital PCR, end-point PCR, multiplex end-point PCR, mass spectrometry, in situ hybridization (ISH), multiplex in situ hybridization, next generation nucleic acid sequencing, or a combination of said methods.

8. The method of claim 7, wherein the next generation nucleic acid sequencing is next generation RNA sequencing, preferably RNA-seq, more preferably single cell RNA-seq or bulk-RNA-seq.

9. A kit for determining the risk to develop complications related to allogeneic hematopoietic stem cell transplantation (allo-HSCT) in a blood sample, the kit comprising means for determining the level of transcription and/or expression and/or activity of the monocyte and/or CTL biomarkers of any one of claims 1 to 8.

10. Use of the method of any one of claims 1 to 8, or the kit of claim 9, for determining the risk to develop complications related to allo-HSCT in a blood sample isolated from a patient transplanted with allo-HSCT.

11. A device for performing a method according to any one of claims 1 to 8, said device comprising:
i) a sample chamber for a test sample collected from a patient transplanted with allo-HSCT;
ii) an assay module in fluid communication with said sample chamber, said assay module comprising means and/or reagents for measuring, directly or indirectly, the expression level of transcription and/or expression and/or activity of the monocyte and/or CTL biomarkers of any one of claims 1 to 8 in said test sample;
iii) means for computing an allo-HSCT probability score; and
iv) a user interface wherein said user interface relates the allo-HSCT probability score to detecting the risk to develop complications related to allo-HSCT.

12. A method of detecting the onset, progression and/ or regression of a complications related to allogeneic hematopoietic stem cell transplantation (allo-HSCT) in a biological sample isolated from a patient transplanted with allo-HSCT, the method comprising:
i) determining the level of transcription and/or expression and/or activity of the monocyte biomarkers SIGLEC1, CD163, and CCL3; and/or
ii) determining the level of transcription and/or expression and/or activity of the cytotoxic T cell (CTL) biomarkers CXCL10 receptor CXCR3 and CD70;
iii) comparing the level of transcription and/or expression and/or activity of said monocyte and/or CTL biomarkers in the transplanted patient blood sample with the level of transcription and/or expression and/or activity in a control sample or with a predetermined reference level,
wherein an increased level of transcription and/or expression and/or activity of said monocyte and/or CTL biomarkers in the transplanted patient sample compared to the control sample or compared to the predetermined reference level is indicative of the onset, progression or regression of said complications related to allo-HSCT.

13. A method of determining the risk to develop complications related to allogeneic hematopoietic stem cell transplantation (allo-HSCT) in a biological sample isolated from a patient transplanted with allo-HSCT, the method comprising:
i) detecting, directly or indirectly, the relative abundance of one or more atypical cell types and/or measuring the level of transcription, expression and/or activity of one or more atypical cell types in a sample obtained from said subject;
ii) periodically comparing the relative abundance and/or level of transcription, expression and/or activity of said one or more atypical cell types,
wherein an alteration in the relative abundance and/or level of transcription, expression and/or activity of one or more of these atypical cell types in a sample obtained from the same subject, relative to the relative abundance and/or level of transcription, expression and/or activity of one or more of these atypical cell types, determined previously, indicates that the patient is at risk to develop complications related to allo-HSCT.

14. A method of detecting the onset, progression and/ or regression of a complications related to allogeneic hematopoietic stem cell transplantation (allo-HSCT) in a biological sample isolated from a patient transplanted with allo-HSCT, said method comprising:
i) detecting, directly or indirectly, the relative abundance of one or more atypical cell types and/or measuring the level of transcription, expression and/or activity of one or more atypical cell types in a sample obtained from said subject;
ii) periodically comparing the relative abundance and/or level of transcription, expression and/or activity of said one or more atypical cell types,
wherein an alteration in the relative abundance and/or level of transcription, expression and/or activity of one or more of these atypical cell types in a sample obtained from the same subject, relative to the relative abundance and/or level of transcription, expression and/or activity of one or more of these atypical cell types, determined previously, is indicative of the onset, progression or regression of said complications related to allo-HSCT .

15. An agent modulating the transcription and/or expression and/or activity
- of a monocyte biomarker selected from the group comprising SIGLEC1, CD163, and CCL3, or a combination of two or more thereof; and/or
- a cytotoxic T cell (CTL) biomarker selected from the group comprising CXCL10 receptor CXCR3 and CD70, or a combination thereof.
